# EUROPEAN PATENT APPLICATION

(11) **EP 4 209 580 A1**
(43) Date of publication of application: **12.07.2023**
(21) Application number: 22185264.3
(22) Date of filing: 15.07.2022
(51) Int. Cl.: C12N 1/20, A23L 27/21, A23L 27/22, A23L 27/23, A23L 27/24, C12P 13/08, C12P 13/10, C12P 13/14, C12P 19/32

(54) **METHOD OF PRODUCING FLAVOR BY MIXED FERMENTATION OF HETEROLOGOUS MICROORGANISMS**

(30) Priority: 11.01.2022 KR 20220004003; 09.06.2022 KR 20220070271
(71) Applicant: Daesang Corporation, Seoul 02586 (KR)
(72) Inventor: KIM, Hyun Ho, 05266 Seoul (KR); LEE, Sun Hee, 16923 Yongin-si (KR); KIM, Dong Hyun, 03472 Seoul (KR); KIM, Hyun Sook, 06622 Seoul (KR); PARK, Seok Hyun, 12258 Namyangju-si (KR); PARK, Joon Hyun, 13523 Seongnam-si (KR)
(74) Representative: Cabinet Nony

(57) **Abstract**

The present invention relates to a method of producing a flavor by a co-fermentation process using mixed fermentation of two or more different microorganisms producing different products. The method of producing a flavor may produce a natural flavor capable of improving the taste and aroma of food and the overall sensory properties of food through a fermentation broth containing amino acids, nucleic acids and/or organic acids, which is produced by mixed fermentation of different microorganisms producing different products, that is, different kinds of amino acids, nucleic acids and/or organic acids. This flavor may be used in various food fields.

## Description

### BACKGROUND

### 1. Technical Field

The present invention relates to a method of producing a flavor by a co-fermentation process using mixed fermentation of two or more different microorganisms producing different products.

### 2. Related Art

L-glutamic acid is an acidic amino acid that gives an umami (or savory) taste, and is naturally present in vegetable foods such as kelp, soybean paste, and soy sauce, as well as animal foods such as dairy products, meat, and fish. This L-glutamic acid is a food flavor that is most frequently used worldwide, and in particular, monosodium glutamate (MSG) produced by adding a salt during purification of L-glutamic acid has an excellent food flavor enhancing effect, and thus is used as a food additive in many processed foods. Recently, as consumer's awareness of health has increased, the market for natural flavors such as yeast extract and natural material extracts has been rapidly growing, but MSG still has an excellent umami taste compared to natural flavors and has high competitiveness at a low price. Therefore, much effort is required to develop a substitute for MSG.

Meanwhile, development of flavors using various amino acids such as L-lysine, L-valine, and L-arginine along with L-glutamic acid has been made for the purpose of enhancing an umami taste. In addition, flavors further comprising nucleic acids such as inosinic acid and guanylic acid, or organic acids such as succinic acid and lactic acid have also been developed. These flavors based on L-glutamic acid are generally produced by mixing fermentation broths produced from microorganisms producing the respective products, or by mixing individually produced amino acid, nucleic acid or organic acid powders. In conventional production methods, it is easy to control the concentration or content of the final product such as L-glutamic acid, but a high production cost is required because the production process must be operated twice before mixing. In addition, large amounts of medium components and fermentation by-products remain in the produced flavor, making it difficult to solve off-flavor and off-odor problems, and there is still a limitation in that it is insufficient to give a rich umami taste. Accordingly, a lot of research and development is needed to develop a flavor with an enhanced umami taste.

### [Prior Art Documents]

### [Patent Documents]

Korean Patent No. 10-1758332
Korean Patent No. 10-1328091

### SUMMARY

An object of the present invention is to provide a method of producing a flavor by mixed fermentation of two or more different microorganisms producing different products.

Another object of the present invention is to provide a method of producing a flavor containing L-glutamic acid and L-lysine by mixed fermentation of a glutamic acid-producing microorganism and a lysine-producing microorganism.

Still another object of the present invention is to provide a flavor produced by the above method.

Yet another object of the present invention is to provide a food composition containing the above flavor.

One aspect of the present invention provides a method for producing a flavor, the method comprising a step of inoculating a fermentation medium with a first microorganism and a second microorganism and then producing a fermentation broth containing amino acid, nucleic acid and/or organic acid by fermentation of the microorganisms, wherein the first microorganism and the second microorganism produce different products and each produces one selected from the group consisting of amino acid, nucleic acid and organic acid.

As used herein, the term "flavor" refers to a substance that is added to enhance the flavor of food, and it is used in a broader sense than a general seasoning that is added in a small amount at the end of a general food cooking process. The flavor may be used to enhance the taste in the process of manufacturing processed foods such as ham, sausage and ramen, as well as when cooking at home. The term "flavor" in the present invention refers to a substance having a richer umami taste and excellent sensory properties by containing one or more amino acids, nucleic acids and/or organic acids as taste components.

According to one embodiment of the present invention, the amino acid may be at least one selected from the group consisting of L-glutamic acid, L-alanine, L-valine, L-leucine, L-isoleucine, L-proline, L-phenylalanine, L-tryptophan, L-methionine, L-glycine, L-serine, L-threonine, L-cysteine, L-asparagine, L-glutamine, L-aspartic acid, L-lysine, L-arginine, and L-histidine.

According to one embodiment of the present invention, the nucleic acid may be at least one selected from the group consisting of inosinic acid, guanylic acid, xanthylic acid, and salts thereof.

For example, the nucleic acid may be inosine monophosphate (IMP), guanosine monophosphate (GMP), xanthosine monophosphate (XMP), etc., without being limited thereto.

According to one embodiment of the present invention, the organic acid may be at least one selected from the group consisting of succinic acid, malic acid, citric acid, acetic acid, lactic acid, fumaric acid, tartaric acid, ascorbic acid, gluconic acid, and salts thereof.

As used herein, the term "fermentation" is a biological phenomenon in which organic matter contained in a medium is decomposed or changed into other substances by microorganisms, and means that the inoculated microorganisms decompose or convert nutrients in the fermentation medium into amino acids, nucleic acids, organic acids, etc.

As used herein, the term "fermentation broth" refers to a broth containing substances produced from microorganisms through fermentation. This fermentation broth is obtained by mixed fermentation of two or more types of microorganisms producing different products, and contains not only useful substances, such as amino acids, nucleic acids and organic acids, produced from each microorganism, but also by-products produced in the metabolic process and medium components.

As used herein, the term "mixed fermentation" refers to a process of fermenting different (or heterologous) microorganisms, which produce different products, that is, different kinds of amino acids, nucleic acids, or organic acids, in a microbial fermentation process in a single fermenter under the same conditions.

The mixed fermentation may be performed using microorganisms producing different kinds of amino acids, nucleic acids or organic acids, or using a combination of an amino acid-producing microorganism and a nucleic acid-producing microorganism, a combination of an amino acid-producing microorganism and an organic acid-producing microorganism, or a combination of an organic acid-producing microorganism and a nucleic acid-producing microorganism, but the present invention is not limited thereto.

The fermentation broth produced by this mixed fermentation may contain two kinds of amino acids, nucleic acids or organic acids, or contain one kind of amino acid and one kind of nucleic acid, one kind of amino acid and one kind of organic acid, or one kind of organic acid and one type of nucleic acid, but the present invention is not limited thereto.

In one example, when the first microorganism is a glutamic acid-producing microorganism, the second microorganism may be a lysine-producing microorganism, an arginine-producing microorganism, a histidine-producing microorganism, a tryptophan-producing microorganism, a glycine-producing microorganism, an alanine-producing microorganism, a succinic acid-producing microorganism , a lactic acid-producing microorganism, a guanylic acid-producing microorganism, or an inosinic acid-producing microorganism.

In addition, when the first microorganism is an inosinic acid-producing microorganism, the second microorganism may be a lysine-producing microorganism, an arginine-producing microorganism, a histidine-producing microorganism, a tryptophan-producing microorganism, a glycine-producing microorganism, an alanine-producing microorganism, a succinic acid-producing microorganism, a lactic acid-producing microorganism, or a guanylic acid-producing microorganism.

According to one embodiment of the present invention, the step may comprise further inoculating the fermentation medium with a third microorganism, which produces a product different from products produced from the first microorganism and the second microorganism and produces one selected from the group consisting of amino acids, nucleic acids and organic acids.

More specifically, the fermentation broth produced from the first microorganism, the second microorganism and the third microorganism contains three kinds of amino acids, nucleic acids or organic acids, or may contain one kind of amino acid and two kinds of nucleic acids, two kinds of amino acids and one kind of nucleic acid, one kind of amino acid and two kinds of organic acids, two kinds of amino acids and one kind of organic acid, one kind of organic acid and two kinds of nucleic acids, two kinds of organic acids and one kind of nucleic acid, or one kind of amino acid, one kind of nucleic acid and one kind of organic acid, but the present invention is not limited thereto.

For example, when the first microorganism is a glutamic acid-producing microorganism and the second microorganism is a guanylic acid-producing microorganism, the third microorganism may be an inosinic acid-producing microorganism.

These microorganisms that are used for the production of a flavor may be microorganisms producing amino acids, nucleic acids and/or organic acids, and may be naturally occurring wild-type microorganisms, or mutant strains modified to improve the production ability of the wild-type microorganisms. As the microorganisms producing amino acids, nucleic acids or organic acids, microorganisms known in the art may be used without limitation, and may be, for example, microorganisms of the genus *Corynebacterium,* the genus *Brevibacterium,* the genus *Lactobacillus,* the genus of *Bifidobacterium,* the genus *Bacillus* spp., etc. These microorganisms may be of the same genus or species, or may be of different genera or species, and may be selected by a user.

According to one embodiment of the present invention, the first microorganism, the second microorganism and the third microorganism may be microorganisms of the genus *Corynebacterium.*

More specifically, the microorganisms of the genus *Corynebacterium* may be *Corynebacterium glutamicum, Corynebacterium crudilactis, Corynebacterium deserti, Corynebacterium callunae, Corynebacterium suranareeae, Corynebacterium lubricantis, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacaterium pseudopelargi* or *Corynebacterium flavescens,* without being limited thereto.

In order to perform mixed fermentation of these two or more microorganisms, isolated and selected microorganisms may be used individually, or a microbial mixture obtained by mixing these microorganisms may be used, and in some cases, an appropriate mixture of the isolated microorganisms and the microbial mixture may be used. Each of the microorganisms may be used for fermentation in a state in which it has been grown and the ability thereof to produce amino acids, nucleic acids and/or organic acids has been activated. In addition, each of the microorganisms is preferably subjected to seed culture for microbial activation.

According to one embodiment of the present invention, the first microorganism, the second microorganism and the third microorganism may be in a seed culture broth state obtained by individual culture or co-culture.

As used herein, the term "seed culture" means culturing a microorganism(s) in a small volume of medium before mass-culturing the microorganism, and the term "seed culture broth" refers to a culture containing medium components, a microorganism proliferated through seed culture, and its metabolites.

The seed culture may be performed using an appropriate medium and culture conditions known in the art in consideration of the characteristics of each microorganism, and those skilled in the art can easily adjust the medium and culture conditions for use.

More specifically, the medium used for seed culture may contain nutrients necessary for microbial growth and proliferation, and may be a liquid medium.

The culture temperature in the seed culture may be usually 20 to 45°C, for example, 25 to 40°C, or 27 to 37°C, and the culture may be continued until each microorganism actively grows and proliferate. The culture time may be, for example, 10 to 160 hours, 18 to 120 hours, or 20 to 80 hours.

In addition, during culture, compounds such as sodium hydroxide, ammonium hydroxide, potassium hydroxide, ammonia, phosphoric acid and sulfuric acid may be added to the medium or culture broth in an appropriate manner to adjust the pH of the culture broth. In addition, it is possible to suppress the formation of air bubbles by using an antifoaming agent for food during culture. Additionally, in order to maintain the medium or culture broth in an aerobic state, oxygen or oxygen-containing gas (e.g., air) may be injected into the culture broth.

This seed culture may be performed depending on a microbial concentration desired by the user, and the optical density (OD) value of the seed culture broth is measured to predict the microbial concentration and determine whether to continue culture.

According to one embodiment of the present invention, the seed culture broth may have OD₆₁₀ = 10 to 80.

In the step of producing a fermentation broth by fermentation of two or more different microorganisms, a fermentation broth containing different products may be produced by mixed fermentation (main fermentation) using different microorganisms or seed culture broths thereof.

The mixed fermentation is a concept opposite to a conventional individual fermentation process of producing a fermentation broth containing the product of each microorganism by culturing each microorganism individually.

In the present invention, through mixed fermentation, for example, it is possible to produce L-glutamic acid by a glutamic acid-producing microorganism, L-lysine by a lysine-producing microorganism, L-arginine by an arginine-producing microorganism, and IMP by an inosinic acid-producing microorganism. In addition, it is possible to produce a fermentation broth capable of exhibiting a rich umami taste and excellent sensory properties due to the interaction between these products and due to taste components such as ions, nucleic acids, organic acids, and other peptides, which are additionally produced in the microbial fermentation process.

According to one embodiment of the present invention, in the step, it is preferable to adjust the inoculum of each microorganism in order to control the ratio between the products of the microorganisms, that is, amino acids, nucleic acids and/or organic acids, in the fermentation broth.

More specifically, when the fermentation medium is inoculated with two different microorganisms, the inoculation ratio between the first microorganism and the second microorganism may be a ratio of 0.05 to 99.95 (first microorganism): 99.95 to 0.05 (second microorganism) relative to the total inoculum. When the fermentation medium is inoculated with three different microorganisms, the inoculation ratio between the first microorganism, the second microorganism and the third microorganism may be 0.05 to 99.95 (first microorganism): 99.95 to 0.05 (second microorganism): 99.95 to 0.05 (third microorganism) relative to the total inoculum.

For example, when the inoculation ratio between the glutamic acid-producing microorganism and the lysine-producing microorganism is 50 to 99.95: 50 to 0.05, L-glutamic acid and L-lysine in the fermentation broth may be produced at a ratio of 0.83 to 99: 1 through mixed fermentation of the two microorganisms so that they can give an appropriate taste as a flavor. When the inoculation ratio between the glutamic acid-producing microorganism and the arginine-producing microorganism is 30 to 99.95: 70 to 0.05, L-glutamic acid and L-arginine in the fermentation broth may be produced at a ratio of 1.04 to 99: 1 through mixed fermentation of the two microorganisms. When the inoculation ratio between the glutamic acid-producing microorganism and the inosinic acid-producing microorganism is 0.05 to 99.95: 99.95 to 0.05, L-glutamic acid and IMP in the fermentation broth may be produced at a ratio of 0.02 to 99.8: 1 through mixed fermentation of the two microorganisms. When the inoculation ratio between the inosinic acid-producing microorganism and the lysine-producing microorganism is 0.05 to 99.95: 99.95 to 0.05, IMP and L-lysine in the fermentation broth may be produced at a ratio of 0.01 to 92.3: 1 through mixed fermentation of the two microorganisms. When the inoculation ratio between the inosinic acid-producing microorganism and the arginine-producing microorganism is 65 to 99.95: 35 to 0.05, IMP and L-arginine in the fermentation broth may be produced at a ratio of 1.04 to 95.1: 1 through mixed fermentation of the two microorganisms.

The mixed fermentation may be performed using an appropriate medium and fermentation conditions known in the art in consideration of the characteristics of each microorganism, and those skilled in the art may easily adjust the medium and fermentation conditions for use.

More specifically, the fermentation medium used for the mixed fermentation may contain nutrients necessary for microbial growth and proliferation, and may be a liquid medium.

The fermentation medium is a medium used during the main fermentation for mass production of amino acids, nucleic acids and/or organic acids, and contains nutrients necessary for the growth of each microorganism. In the present invention, since the fermentation broth containing the fermentation medium is used as a flavor without a separate purification process after completion of the fermentation, it is preferable that the fermentation medium is composed of substances usable as food substances and contains the minimum components and amounts required for culturing microorganisms.

According to one embodiment of the present invention, the fermentation medium may be based on molasses and contain raw sugar and/or glucose.

More specifically, the fermentation medium may contain molasses, raw sugar, and glucose as both sugar sources and nutrient sources for microorganisms, and may contain molasses in an amount of 1 to 30 wt% based on the total sugar amount. Molasses used herein may be derived from sugar cane or sugar beets.

The fermentation medium may further contain nutrients for nutrient enhancement of microorganisms, in addition to the sugar sources.

According to one embodiment of the present invention, the fermentation medium may further contain at least one selected from the group consisting of yeast extract, phosphoric acid, and betaine.

The fermentation temperature in the mixed fermentation may be usually 20 to 45°C, for example, 25 to 40°C, or 30 to 38°C, and the fermentation may be continued until the microorganisms actively grow and proliferate. The fermentation time may be, for example, 10 to 160 hours, 18 to 120 hours, or 20 to 100 hours.

In addition, during culture, compounds such as sodium hydroxide, ammonium hydroxide, potassium hydroxide, ammonia, phosphoric acid and sulfuric acid may be added to the medium or culture broth in an appropriate manner to adjust the pH of the culture broth. In addition, it is possible to suppress the formation of air bubbles by adding an antifoaming agent for food during culture. Additionally, in order to maintain the medium or culture broth in an aerobic state, oxygen or oxygen-containing gas (e.g., air) may be injected into the culture broth.

The fermentation broth produced through this mixed fermentation is used as the raw material of a flavor, is characterized by being used intact without being mixed with additional components, and contains large amounts of amino acids, nucleic acids and/or organic acids produced from the microorganisms.

According to one embodiment of the present invention, the fermentation broth may contain total microbial products in an amount of 3 to 90 wt% based on the total solid content.

More specifically, the fermentation broth may contain amino acids, nucleic acids and/or organic acids in an amount of 3 to 90 wt%, 10 to 90 wt%, 20 to 90 wt%, 30 to 90 wt%, 40 to 90 wt%, 50 to 90 wt%, 60 to 90 wt%, 70 to 90 wt%, or 80 to 90 wt%, based on the total solid content. This fermentation broth may contain 5 to 150 g/L of amino acids, nucleic acids and/or organic acids.

Another aspect of the present invention provides a method for producing a flavor containing L-glutamic acid and L-lysine, the method comprising a step of inoculating a fermentation medium with a glutamic acid-producing microorganism and a lysine-producing microorganism and then producing a fermentation broth containing L-glutamic acid and L-lysine by fermentation of the microorganisms.

The glutamic acid-producing microorganism and the lysine-producing microorganism may be naturally occurring wild-type microorganisms or mutant strains modified to improve the amino acid production ability of the wild-type microorganisms. As this glutamic acid- or lysine-producing microorganism, a microorganism known in the art may be used without limitation. For example, the glutamic acid- or lysine-producing microorganism may be a microorganism of the genus *Corynebacterium,* the genus *Brevibacterium,* the genus *Lactobacillus,* the genus *Bifidobacterium,* the genus *Bacillus,* etc. The microorganisms in the present invention may be of the same genus or species, or different genera or species, and may be selected by the user.

According to one embodiment of the present invention, the glutamic acid-producing microorganism and the lysine-producing microorganism may be microorganisms of the genus *Corynebacterium.*

In an example of the present invention, *Corynebacterium glutamicum* strains were used as the glutamic acid-producing microorganism and the lysine-producing microorganism.

In order to perform mixed fermentation of the glutamic acid-producing microorganism and the lysine-producing microorganism, isolated and selected microorganisms may be used individually, or a microbial mixture obtained by mixing these microorganisms may be used, and in some cases, an appropriate mixture of the isolated microorganisms and the microbial mixture may be used. This glutamic acid-producing microorganism or lysine-producing microorganism may be used for fermentation in a state in which it has been grown and the ability thereof to produce amino acids has been activated. In addition, each of the microorganisms is preferably subjected to seed culture for microbial activation.

According to one embodiment of the present invention, the glutamic acid-producing microorganism and lysine-producing microorganism may be in a seed culture broth state obtained by individual culture or co-culture.

The seed culture may be performed using an appropriate medium and culture conditions known in the art in consideration of the characteristics of each microorganism, and any person skilled in the art can easily adjust the medium and culture conditions for use.

More specifically, the medium used for the seed culture may contain nutrients necessary for microbial growth and proliferation, and may be a liquid medium.

According to one embodiment of the present invention, the medium for seed culture of the glutamic acid-producing microorganism may contain, based on the total weight of the medium, 4.5 to 5.5 wt% of molasses, 3 wt% of glucose, 0.85 wt% of yeast extract paste, 100 ppm of methionine, 0.6 wt% of H₃PO₄, 0.1 wt% of sodium succinate, 50 ppm of vitamin C, 12 ppm of thiamine HCl, 20 ppb of vitamin B12, 10 ppm of biotin, 0.4 wt% of MgSO₄, and 0.01 wt% of an antifoaming agent for food.

According to one embodiment of the present invention, the medium for seed culture of the lysine-producing microorganism may contain, based on the total weight of the medium, 1.5 to 3 wt% of molasses, 9 to 12 wt% of raw sugar, 1 wt% of yeast extract paste, 1.6 wt% of (NH₄)₂SO₄, 0.3 wt% of H₃PO₄, 7.3 ppm of MnSO₄·5H₂O, 14 ppm of nicotinamide, 2.5 ppm of thiamin HCl, 1.5 ppm of CuSO₄·5H₂O, 0.056 ppm of biotin, 0.045 wt% of betaine, and 0.01 wt% of an antifoaming agent for food.

The culture temperature in the seed culture may be usually 20 to 45°C, for example, 25 to 40°C, or 27 to 37°C, and the culture may be continued until each microorganism actively grows and proliferates. The culture time may be, for example, 10 to 160 hours, 18 to 120 hours, or 20 to 80 hours.

In addition, during culture, compounds such as sodium hydroxide, ammonium hydroxide, potassium hydroxide, ammonia, phosphoric acid and sulfuric acid may be added to the medium or culture broth in an appropriate manner to adjust the pH of the culture broth. In addition, it is possible to suppress the formation of air bubbles by using an antifoaming agent for food during culture. Additionally, in order to maintain the medium or culture broth in an aerobic state, oxygen or oxygen-containing gas (e.g., air) may be injected into the culture broth.

This seed culture may be performed depending on a microbial concentration desired by the user, and the optical density (OD) value of the seed culture broth is measured to predict the microbial concentration and determine whether to continue culture.

According to one embodiment of the present invention, the seed culture broth may have OD₆₁₀ = 10 to 80.

In the step of producing a fermentation broth by fermentation of the glutamic acid-producing microorganism and the lysine-producing microorganism, a fermentation broth containing both L-glutamic acid and L-lysine may be produced by mixed fermentation (main fermentation) using the glutamic acid-producing microorganism or a seed culture broth thereof and the lysine-producing microorganism or a seed culture broth thereof.

The mixed fermentation is a concept opposite to a conventional individual fermentation process of producing a fermentation broth containing the product of each microorganism by culturing each microorganism individually for amino acid production.

In the present invention, through mixed fermentation, it is possible to produce L-glutamic acid by the glutamic acid-producing microorganism and L-lysine by the lysine-producing microorganism. In addition, it is possible to produce a fermentation broth capable of exhibiting a rich umami taste and excellent sensory properties due to the interaction between L-glutamic acid and L-lysine and due to taste components such as ions, nucleic acids, organic acids, and other peptides, which are additionally produced in the microbial fermentation process.

According to one embodiment of the present invention, in the step, it is preferable to adjust the inoculum of each of the glutamic acid-producing microorganism and the lysine-producing microorganism in order to control the ratio between L-glutamic acid and L-lysine in the fermentation broth.

More specifically, the inoculation ratio between the glutamic acid-producing microorganism and the lysine-producing microorganism may be a ratio of 0.05 to 99.95 (glutamic acid-producing microorganism): 99.95 to 0.05 (lysine-producing microorganism) relative to the total inoculum. Through mixed fermentation of the two microorganisms inoculated at this ratio, L-glutamic acid and L-lysine in the fermentation broth may be produced at a ratio of 1 to 99: 1 so that they may give an appropriate taste as a flavor.

For example, when the inoculation ratio between the glutamic acid-producing microorganism and the lysine-producing microorganism is 50 to 99.95: 50 to 0.05, L-glutamic acid and L-lysine in the fermentation broth may be produced at a ratio of 0.83 to 99: 1, and when the inoculation ratio between the glutamic acid-producing microorganism and the lysine-producing microorganism is adjusted to 65: 35, L-glutamic acid and L-lysine in the fermentation broth may be produced at a ratio of about 1: 1.

The mixed fermentation may be performed using an appropriate medium and fermentation conditions known in the art in consideration of the characteristics of each microorganism, and any person skilled in the art may easily adjust the medium and fermentation conditions for use.

More specifically, the fermentation medium used for the mixed fermentation may be a liquid medium.

The fermentation medium is a medium used during the main fermentation for mass production of L-glutamic acid and L-lysine, and contains nutrients necessary for the growth of the glutamic acid-producing microorganism and the lysine-producing microorganism. In the present invention, since the fermentation broth containing the fermentation medium is used as a flavor without a separate purification process after completion of the fermentation, it is preferable that the fermentation medium is composed of substances usable as food substances and contains the minimum components and amounts required for culturing the microorganisms.

According to one embodiment of the present invention, the fermentation medium may be based on molasses and contain raw sugar and/or glucose.

More specifically, the fermentation medium may contain molasses, raw sugar, and glucose as both sugar sources and nutrient sources for the microorganisms, and may contain molasses in an amount of 1 to 30 wt% based on the total sugar amount. Molasses used herein may be derived from sugar cane or sugar beets.

The fermentation medium may further contain nutrients for nutrient enhancement of microorganisms, in addition to the sugar sources.

According to one embodiment of the present invention, the fermentation medium may further contain at least one selected from the group consisting of yeast extract, phosphoric acid, and betaine.

According to one embodiment of the present invention, the fermentation medium may contain, based on the total weight of the medium, 1.5 to 3 wt% of molasses, 2.5 to 4 wt% of glucose, 0.4 to 1 wt% of yeast extract paste, 0.1 to 0.2 wt% of H₃PO₄, 0.05 to 0.12 wt% of betaine, and 0.001 to 0.01 wt% of an antifoaming agent for food.

The fermentation temperature in the mixed fermentation may be usually 20 to 45°C, for example, 25 to 40°C, or 30 to 38°C, and the fermentation may be continued until the contents or concentrations of L-glutamic acid and L-lysine obtained reach desired levels. The fermentation time may be, for example, 10 to 160 hours, 18 to 120 hours, or 20 to 100 hours.

In addition, during fermentation, compounds such as sodium hydroxide, ammonium hydroxide, potassium hydroxide, ammonia, phosphoric acid and sulfuric acid may be added to the medium or fermentation broth in an appropriate manner to adjust the pH of the fermentation broth. In addition, it is possible to suppress the formation of air bubbles by using an antifoaming agent for food during fermentation. Additionally, in order to maintain the medium or fermentation broth in an aerobic state, oxygen or oxygen-containing gas (e.g., air) may be injected into the fermentation broth.

According to one embodiment of the present invention, the fermentation may be performed by fed-batch culture, which starts at a temperature of 30 to 33°C and is maintained at a temperature of 36 to 39°C, a pH of 6.5 to 7.5 and a dissolved oxygen concentration of 20 to 70%, for 28 to 40 hours.

This fermentation broth containing L-glutamic acid and L-lysine is used as the raw material of a flavor containing L-glutamic acid and L-lysine, is characterized by being used intact without being mixed with additional components, and contains large amounts of L-glutamic acid and L-lysine.

According to one embodiment of the present invention, the fermentation broth may contain amino acids, including L-glutamic acid and L-lysine, in an amount of 3 to 90 wt% based on the total solid content.

More specifically, the fermentation broth may contain amino acids, including L-glutamic acid and L-lysine, in an amount of 3 to 90 wt%, 10 to 90 wt%, 20 to 90 wt%, 30 to 90 wt%, 40 to 90 wt%, 50 to 90 wt%, 60 to 90 wt%, 70 to 90 wt%, or 80 to 90 wt%, based on the total solid content. This fermentation broth may contain 5 to 150 g/L of L-glutamic acid and L-lysine.

Meanwhile, the method for producing a flavor according to the present invention may further comprise an additional process for using the fermentation broth as a flavor or a flavor containing L-glutamic acid and L-lysine.

More specifically, the method may further comprise a step of separating cells from the fermentation broth and decolorizing the fermentation broth.

The cell separation may be performed without limitation by applying a cell separation method and separation conditions known in the art. An example of the cell separation method may be membrane separation, ultrafiltration, centrifugal filtration, or the like, but is not limited thereto.

The decolorization may be performed without limitation by applying a decolorization method and decolorization conditions known in the art. For example, the decolorization may be performed using activated carbon or the like, without being limited thereto.

In addition, the method may further comprise a step of filtering the decolorized fermentation broth.

The filtration may be performed without limitation by applying a filtration method and filtration conditions known in the art. For example, the filtration may be performed using filter paper, filter net, membrane filtration, ultrafiltration, etc., without being limited thereto.

In addition, the method may further comprise a step of concentrating the filtered fermentation broth.

The concentration may be performed without limitation by applying a concentration method and concentration conditions known in the art. Examples of the concentration method include, but are not limited to, heat concentration, vacuum concentration, freeze concentration, evaporation concentration, vacuum low-temperature concentration, and the like.

In addition, the method may further comprise a step of drying and powdering the concentrated fermentation broth.

The drying may be performed without limitation by applying a drying method and drying conditions known in the art. Examples of the drying method include, but are not limited to, freeze drying, vacuum drying, air drying, blown air drying, hot air drying, fluidized bed drying, spray drying, infrared drying, high frequency drying, and the like.

Through this method, the fermentation broth may be finally obtained in a powder state, and may be used as a natural food flavor without undergoing an additional chemical purification process.

Still another aspect of the present invention provides a flavor produced by the above-described method for producing a flavor using two or more different microorganisms.

According to one embodiment of the present invention, the flavor may be a natural flavor containing two or three taste components selected from among amino acid, nucleic acid and organic acid.

The amino acid may be at least one selected from the group consisting of L-glutamic acid, L-alanine, L-valine, L-leucine, L-isoleucine, L-proline, L-phenylalanine, L-tryptophan, L-methionine, L-glycine, L-serine, L-threonine, L-cysteine, L-asparagine, L-glutamine, L-aspartic acid, L-lysine, L-arginine, and L-histidine.

The nucleic acid may be at least one selected from the group consisting of inosinic acid, guanylic acid, xanthyl acid, and salts thereof.

For example, the nucleic acid may be inosine monophosphate (IMP), guanosine monophosphate (GMP), xanthosine monophosphate (XMP), etc., but is not limited thereto.

The organic acid may be at least one selected from the group consisting of succinic acid, malic acid, citric acid, acetic acid, lactic acid, fumaric acid, tartaric acid, ascorbic acid, gluconic acid, and salts thereof.

For example, when the flavor contains two taste components, it may contain glutamic acid and lysine, arginine, histidine, tryptophan, glycine, alanine, succinic acid, lactic acid, guanylic acid, or inosinic acid. In addition, the flavor may contain inosinic acid and lysine, arginine, histidine, tryptophan, glycine, alanine, succinic acid, lactic acid, or guanylic acid.

In another example, when the flavor contains three taste components, it may contain glutamic acid, guanylic acid, and inosinic acid.

According to one embodiment of the present invention, the flavor may contain taste components, including amino acids, nucleic acids and/or organic acids, in an amount of 3 to 90 wt% based on the total solid content.

More specifically, the flavor may contain taste components, including amino acids, nucleic acids and/or organic acids, in an amount of 3 to 90 wt%, 10 to 90 wt%, 20 to 90 wt%, 30 to 90 wt%, 40 to 90 wt%, 50 to 90 wt%, 60 to 90 wt%, 70 to 90 wt%, or 80 to 90 wt%, based on the total solid content.

Yet another aspect of the present invention provides a flavor containing L-glutamic acid and L-lysine, produced by the above-described method for producing a natural flavor containing L-glutamic acid and L-lysine using a glutamic acid-producing microorganism and a lysine-producing microorganism.

According to one embodiment of the present invention, the flavor containing L-glutamic acid and L-lysine may be a natural flavor.

According to one embodiment of the present invention, the flavor may have an L-glutamic acid and L-lysine content of 3 to 90 wt% based on the solid content, and contain L-glutamic acid and L-lysine at a ratio of 0.83 to 99: 1.

Since this flavor contains L-lysine together with L-glutamic acid that gives an umami taste, it may increase the solubility of L-glutamic acid and solve the ammonia odor problem by reducing the production of ammonium glutamate during the fermentation process. In addition, since the flavor also contains metabolites such as organic acids, inorganic ion components, proteins, peptides, and vitamins produced in the fermentation process, it has a rich umami taste and a strong body feeling and exhibits excellent sensory properties. Thus, the flavor may be added to various foods to maximize the taste of the foods.

Still yet another aspect of the present invention provides a food composition containing the above-described flavor or the flavor containing L-glutamic acid and L-lysine.

As used herein, the term "food composition" refers to a natural product or processed product containing one or more nutrients, and preferably refers to a product that has undergone certain processing and is ready to be eaten directly. In a common sense, the term "food composition" includes all health functional foods, functional foods, beverages, food additives, and beverage additives.

According to one embodiment of the present invention, the food composition contains a flavor produced without a chemical purification process or a flavor containing natural L-glutamic acid and L-lysine, that is a natural flavor, and it may contain the flavor in an amount of 0.001 to 90 wt%, more specifically 0.01 to 50 wt%, based on the total weight of the composition.

The food composition in the present invention may be provided in any formulation suitable for food, and may be, for example, in the form of a solution, emulsion, viscous mixture, powder, granule, tablet, capsule, or the like. In this case, the food composition may contain various bases and/or additives necessary and appropriate for formulation within the range that does not impair the main effect of the present invention. In addition, the food composition may further contain additives such as fragrance, colorants, disinfectants, antioxidants, preservatives, humectants, thickeners, inorganic salts, and emulsifiers, within the range that does not impair the effect thereof. The content of these additives in the food composition may be selected depending on the formulation or intended use of the composition within the range that does not impair the purpose and effect of the present invention. For example, the content of the additives may be 0.01 to 70 wt%, more specifically 0.1 to 50 wt%, based on the total weight of the food composition.

This food composition may be used as an additive for various foods. As foods to which this food composition may be added, any foods known in the art may be used without limitation. Examples of the foods include, but are not limited to, meat, sausage, bread, chocolate, candy, snacks, confectionery, pizza, ramen, other noodles, gums, dairy products including ice cream, various soups, beverages, tea, drinks, alcoholic beverages, and vitamin complexes.

The method for producing a flavor according to the present invention may produce a natural flavor capable of improving the taste and aroma of food and the overall sensory properties of food through a fermentation broth containing amino acids, nucleic acids and/or organic acids, which is produced by mixed fermentation of different microorganisms producing different products, that is, different kinds of amino acids, nucleic acids and/or organic acids. This flavor may be used in various food fields.

### BRIEF DESCRIPTION OF THE DRAWINGS

FIG. 1 is a flow chart showing a fermentation process performed using a glutamic acid-producing microorganism and a lysine-producing microorganism in Preparation Examples 1 to 3 according to one embodiment of the present invention.
FIG. 2 is a flow chart showing a fermentation process performed using a glutamic acid-producing microorganism and an arginine-producing microorganism in Preparation Examples 4 to 6 according to one embodiment of the present invention.
FIG. 3 is a flow chart showing a fermentation process performed using a glutamic acid-producing microorganism and an inosinic acid-producing microorganism in Preparation Examples 7 to 9 according to one embodiment of the present invention.
FIG. 4 is a flow chart showing a fermentation process performed using an inosinic acid-producing microorganism and a lysine-producing microorganism in Preparation Examples 10 to 12 according to one embodiment of the present invention.
FIG. 5 is a flow chart showing a fermentation process performed using an inosinic acid-producing microorganism and an arginine-producing microorganism in Preparation Examples 13 to 15 according to one embodiment of the present invention.

### DETAILED DESCRIPTION

Hereinafter, the present invention will be described in more detail with reference to the accompanying drawings. However, these descriptions are provided for illustrative purposes only to help the understanding of the present invention, and the scope of the present invention is not limited by these illustrative descriptions.

### Example 1. Mixed fermentation of glutamic acid and lysine

### 1-1. Seed culture

*Corynebacterium glutamicum* NFG6 (KCCM13164P) that produces L-glutamic acid (GA) was used as a glutamic acid-producing microorganism, and *Corynebacterium glutamicum* NFL21 (KCCM13163P) that produces L-lysine (LYS) was used as a lysine-producing microorganism.

For seed culture of the glutamic acid-producing microorganism, the glutamic acid-producing microorganism was inoculated into a 2-L flask containing 0.2 L of a seed culture medium, followed by primary culture for 22 to 24 hours at 30°C and 140 rpm (OD₆₁₀ = 10 to 15). Next, 2 to 3% of the primary culture was inoculated into a 5-L jar fermenter, and 2 to 2.5 L of a seed culture medium was added thereto, followed by secondary culture at 32°C, pH 6.9, 600 rpm and an aeration rate of 1.0 vvm for 22 to 24 hours (OD₆₁₀ = 20 to 60), thereby preparing a glutamic acid seed culture broth.

For seed culture of the lysine-producing microorganism, the lysine-producing microorganism was inoculated into a 2-L flask containing 0.2 L of a seed culture medium, followed by primary culture for 16 to 18 hours at 30°C and 140 rpm (OD₆₁₀ = 11 to 12). Next, 5% of the primary culture was inoculated into a 5-L jar fermenter, and 2 to 2.5 L of a seed culture medium was added thereto, followed by secondary culture at 32°C, pH 7.0, 650 rpm and an aeration rate of 2.0 vvm for 21 to 24 hours (OD₆₁₀ = 20 to 60), thereby preparing a lysine seed culture broth.

The compositions of the seed culture media used are shown in Table 1 below.

**[Table 1]**

| | Composition |
|---|---|
| Seed culture medium for glutamic acid-producing microorganism | 4.5 to 5.5% molasses, 3% glucose, 0.85% yeast extract paste, 100 ppm methionine, 0.6% H₃PO₄, 0.1% sodium succinate, 50 ppm vitamin C, 12 ppm thiamine HCl, 20 ppb vitamin B12, 10 ppm biotin, 0.4% MgSO₄, and 0.01% antifoaming agent for food |
| Seed culture medium for lysine-producing microorganism | 1.5 to 3% molasses, 9 to 12% raw sugar, 1% yeast extract paste, 1.6% (NH₄)₂SO₄, 0.3% H₃PO₄, 7.3 ppm MnSO₄·5H₂O, 14 ppm nicotinamide, 2.5 ppm thiamine HCl, 1.5 ppm CuSO₄·5H₂O, 0.056 ppm biotin, 0.045% betaine, and 0.01% antifoaming agent for food |

### 1-2. Main fermentation

In order to examine the ratio of glutamic acid to lysine in the fermentation broth depending on the inoculums of the glutamic acid seed culture broth and the lysine seed culture broth, the glutamic acid seed culture broth and the lysine seed culture broth were inoculated at various ratios and fermented.

In main fermentation, 14 to 18 L of a fermentation medium was added to a 50 L fermenter, and inoculated with 1.2 to 1.8 L of the total seed culture broth at an inoculation ratio of 50 to 99.95 (glutamic acid seed culture broth): 50 to 0.05 (lysine seed culture broth), followed by mixed fermentation by fed-batch culture for 28 to 40 hours. The fermentation medium composition and fermentation conditions used are shown in Table 2 below.

**[Table 2]**

| | |
|---|---|
| Fermentation medium composition | 1.5 to 3% molasses, 2.5 to 4% glucose, 0.4 to 1% yeast extract paste, 0.1 to 0.2% H₃PO₄, 0.05 to 0.12% betaine, and 0.005% antifoaming agent for food |
| Fermentation conditions | Temperature 32→38°C, pH 6.5 to 7.5, aeration rate 0.8 to 1.2 vvm, internal pressure 0.6 to 1.0 kg/cm³, agitation speed 320 to 350 rpm, and dissolved oxygen (DO) concentration 20 to 70% |

This mixed fermentation was performed a total of three times, the average value was calculated, and the results are shown in Table 3 below.

**[Table 3]**

| Inoculation ratio between seed culture broths (GA: LYS) | Fermentation time | GA: LYS ratio in fermentation broth |
|---|---|---|
| 99.95 : 0.05 | 31 hours | 99 : 1 |
| 75 : 25 | 29 hours | 1.48 : 1 |
| 70 : 30 | 29 hours | 1.30 : 1 |
| 67 : 33 | 30 hours | 1.10 : 1 |
| 65 : 35 | 30 hours | 1.01 : 1 |
| 60 : 40 | 29 hours | 0.93 : 1 |
| 50 : 50 | 28 hours | 0.83 : 1 |

Referring to Table 3 above, it was confirmed that, when the seed culture broths of the amino acid-producing microorganisms were inoculated at a ratio of 50 to 99.95 (glutamic acid-producing microorganism): 50 to 0.05 (lysine-producing microorganism), L-glutamic acid and L-lysine in the fermentation broth were produced at a ratio of 0.83 to 99: 1.

### Example 2. Comparison of glutamic acid-lysine fermented powders between fermentation processes

### 2-1. Production of glutamic acid-lysine fermented powder

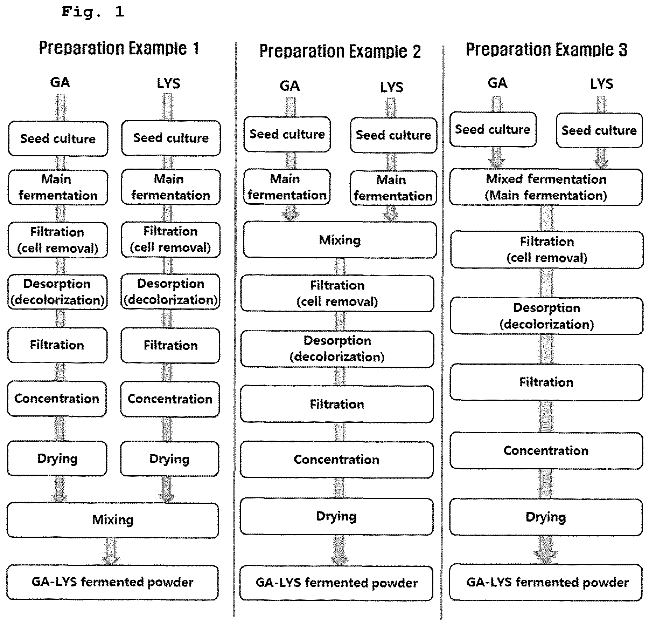

Conventionally, a flavor containing glutamic acid and lysine was produced by individually fermenting a glutamic acid-producing microorganism and a lysine-producing microorganism and then mixing the amino acid-containing fermentation broths or dried products thereof at a suitable ratio. To compare the differences in taste between this conventional individual fermentation method and the method based on mixed fermentation of the glutamic acid-producing microorganism and the lysine-producing microorganism, the components of glutamic acid-lysine (GA-LYS) fermented powders obtained by the production methods were compared. (See FIG. 1).

The glutamic acid seed culture broth and lysine seed culture broth used here were prepared in the same manner as in Example 1-1.

### ① Individual fermentation (Preparation Example 1)

In the method of mixing individual dried products among the conventional methods, each of the glutamic acid seed culture broth and the lysine seed culture broth was transferred into a 50-L fermenter and then individually subjected to main fermentation. Next, cells were separated from each fermentation broth, and then decolorization and filtration processes were performed. The filtrates were concentrated and dried to obtain dried products. The obtained dried glutamic acid and lysine products were mixed together so that the ratio of glutamic acid to lysine was 1:1, thereby preparing fermented powder containing glutamic acid and lysine.

### ② Mixing of fermentation broths after individual fermentation (Preparation Example 2)

In the method of mixing individual fermentation broths among the conventional methods, each of the glutamic acid seed culture broth and the lysine seed culture broth was transferred into a 50-L fermenter and then individually subjected to main fermentation. The fermentation broths obtained in the main fermentation were mixed together so that the ratio of glutamic acid to lysine was 1: 1. Next, cells were separated from the fermentation broth mixture, and then decolorization and filtration processes were performed. The filtrate was concentrated and dried to obtain glutamic acid-lysine fermented powder.

### ③ Mixed fermentation (Preparation Example 3)

In mixed fermentation, the glutamic acid seed culture broth and the lysine seed culture broth were inoculated at a ratio of 65: 35 in the same manner as in Example 1-2 so that the ratio of glutamic acid to lysine in the fermentation broth was about 1:1 in the same manner as in Preparation Examples 1 and 2, followed by mixed fermentation. Next, cells were separated from the fermentation broth, and decolorization and filtration processes were performed. The filtrate was concentrated and dried to obtain glutamic acid-lysine fermented powder.

### 2-2. Comparison of components between glutamic acid-lysine fermented powders

Component analysis was performed on the glutamic acid-lysine fermented powder obtained through individual fermentation in each of Preparation Examples 1 and 2 and the glutamic acid-lysine fermented powder obtained through mixed fermentation in Preparation Example 3.

Amino acids (L-glutamic acid and L-lysine) were measured by HPLC analysis (GA - 210 nm, UV detector, flow rate 0.9 ml/min; LYS - 214 nm, UV detector, flow rate 0.8 ml/min), and organic acids (citric acid, succinic acid, lactic acid, acetic acid, etc.) were measured by HPLC analysis (HPX-87H column, 214 nm, 25 min). Ions (Na, Mg, K, PO₄, SO₄, Cl, NH₄, etc.) were measured by an ion analyzer (Dionex IcS-1100, Thermo Scientific). The results are shown in Table 4 below.

**[Table 4]**

| Item | Preparation Example 1 | Preparation Example 2 | Preparation Example 3 |
|---|---|---|---|
| GA | 22% | 35% | 43% |
| LYS | 22% | 35% | 43% |
| AA/TS* | 47% | 72% | 88% |
| Total nitrogen | 9.0% | 12.2% | 11.8% |
| Organic acids | 3.1% | 2.6% | 1.5% |
| Ions | 8.1% | 10.2% | 2% |
| Ammonium | 3.8% | 2.5% | 0.7% |
| *AA/TS: proportion of amino acids (GA+LYS) relative to total solid content | | | |

Referring to Table 4 above, mixed fermentation (Preparation Example 3) showed a higher amino acid proportion than individual fermentation (Preparation Examples 1 and 2) and showed significant decreases in the contents of organic acids, ions and ammonium.

### 2-3. Sensory comparison between individual fermentation and mixed fermentation

Sensory evaluation was performed on the glutamic acid-lysine fermented powders obtained through individual fermentation in Preparation Examples 1 and 2 and the glutamic acid-lysine fermented powder obtained through mixed fermentation in Preparation Example 3.

For sensory evaluation of the glutamic acid-lysine fermented powders, the umami taste, umami persistence, salty taste, sour taste, bitter taste and sweet taste of each powder itself or a sample obtained by diluting each powder in lukewarm water at a concentration of 1 to 5% were evaluated by a trained panel consisting of 10 to 15 persons. The results are shown in Table 5 below.

**[Table 5]**

| Item | Preparation Example 1 (indi vidual fermentation) | Preparation Example 2 (individual fermentation) | Preparation Example 3 (mixed fermentation) |
|---|---|---|---|
| Initial Umami taste | +++ | ++++ | ++++ |
| Umami persistence | ++ | +++ | ++++ |
| Salty taste | - | + | ++ |
| Sour taste | ++ | + | - |
| Bitter taste | ++ | + | - |
| Sweet taste | + | + | ++ |

Referring to Table 5 above, due to the difference in components of the glutamic acid-lysine fermented powder as shown in Table 4 above, the sample prepared by the mixing process after individual fermentation showed a weak umami taste and an increased bitter taste and sour taste compared to the sample prepared by mixed fermentation. In addition, since the increase in by-products such as organic acids and the increase in ions as shown in Table 4 above also affect the sensory properties of the sample, the mixed fermentation process is more effective in terms of taste or process simplification than mixing after individual fermentation.

### Example 3. Mixed fermentation of glutamic acid and arginine

### 3-1. Seed culture

*Corynebacterium glutamicum* NFG6 (KCCM13164P) was used as a glutamic acid-producing microorganism, and *Corynebacterium glutamicum* NFA40 (KCCM13165P) that produces L-arginine (ARG) was used as an arginine-producing microorganism.

A glutamic acid seed culture broth of the glutamic acid-producing microorganism was prepared in the same manner as in Example 1-1.

For seed culture of the arginine-producing microorganism, the arginine-producing microorganism was inoculated into a 2-L flask containing 0.2 L of a seed culture medium, followed by primary culture for 16 to 18 hours at 30°C and 140 rpm (OD₆₁₀ = 12 to 18). Next, 2 to 4% of the primary culture was inoculated into a 5-L jar fermenter, and 2 to 2.5 L of a seed culture medium was added thereto, followed by secondary culture at 32°C, pH 6.7, 600 rpm and an aeration rate of 1.0 vvm for 16 to 24 hours (OD₆₁₀ = 20 to 60), thereby preparing an arginine seed culture broth. The composition of the seed culture medium used is shown in Table 6 below.

**[Table 6]**

| | Composition |
|---|---|
| Seed culture medium for arginine-producing microorganism | 1.5 to 4.5% glucose, 2 to 6% raw sugar, 2 to 3% yeast extract paste, 0.6% (NH₄)₂SO₄, 0.2% KH₂PO₄, 0.2% K₂HPO₄ 0.2%, 15 ppm MnSO₄·5H₂O, 0.2 % MgSO₄·7H₂O 1 ppm thiamine HCl, 10 ppm ZnSO₄·7H₂O, 0.3 ppm biotin, 15 ppm FeSO₄·7H₂O, and 0.01% antifoaming agent for food |

### 3-2. Main fermentation

In order to examine the ratio of glutamic acid to arginine in the fermentation broth depending on the inoculums of the glutamic acid seed culture broth and the arginine seed culture broth, the glutamic acid seed culture broth and the arginine seed culture broth were inoculated at various ratios and fermented.

In main fermentation, 14 to 18 L of a fermentation medium was added to a 50-L fermenter and inoculated with 1.2 to 1.8 L of the total seed culture broth at an inoculation ratio of 30 to 99.95 (glutamic acid seed culture broth): 70 to 0.05 (arginine seed culture broth), followed by mixed fermentation by fed-batch culture for 31 to 60 hours. The fermentation medium composition and fermentation conditions used are shown in Table 7 below.

**[Table 7]**

| | |
|---|---|
| Fermentation medium composition | 1.5 to 3% molasses, 2.5 to 4% glucose, 0.4 to 1% yeast extract paste, 0.05 to 0.3% (NH₄)₂SO₄, 0.1 to 0.2% H₃PO₄, 0.05 to 0.12% betaine, and 0.005% antifoaming agent for food |
| Fermentation conditions | Temperature 32 to 37°C, pH 6.5 to 7.5, aeration rate 0.8 to 1.2 vvm, internal pressure 0.5 to 1.0 kg/cm³, agitation speed 320 to 350 rpm, and dissolved oxygen (DO) concentration 20 to 70% |

This mixed fermentation was performed a total of three times, the average value was calculated, and the results are shown in Table 8 below.

**[Table 8]**

| Inoculation ratio between seed culture broths (GA: ARG) | Fermentation time | GA: ARG ratio in fermentation broth |
|---|---|---|
| 99.95 : 0.05 | 31 hours | 99.0 : 1 |
| 90 : 10 | 31 hours | 12.2 : 1 |
| 80 : 20 | 34 hours | 4.36 : 1 |
| 70 : 30 | 36 hours | 2.41 : 1 |
| 60 : 40 | 38 hours | 1.96 : 1 |
| 50 : 50 | 40 hours | 1.57 : 1 |
| 40 : 60 | 44 hours | 1.21 : 1 |
| 30 : 70 | 45 hours | 1.04 : 1 |

Referring to Table 8 above, it was confirmed that, when the seed culture broths of the amino acid-producing microorganisms were inoculated at a ratio of 30 to 99.95 (glutamic acid-producing microorganism): 70 to 0.05 (arginine-producing microorganism), L-glutamic acid and L-arginine in the fermentation broth were produced at a ratio of 1.04 to 99: 1.

### Example 4. Comparison of glutamic acid-arginine fermented powders between fermentation processes

### 4-1. Production of glutamic acid-arginine fermented powder

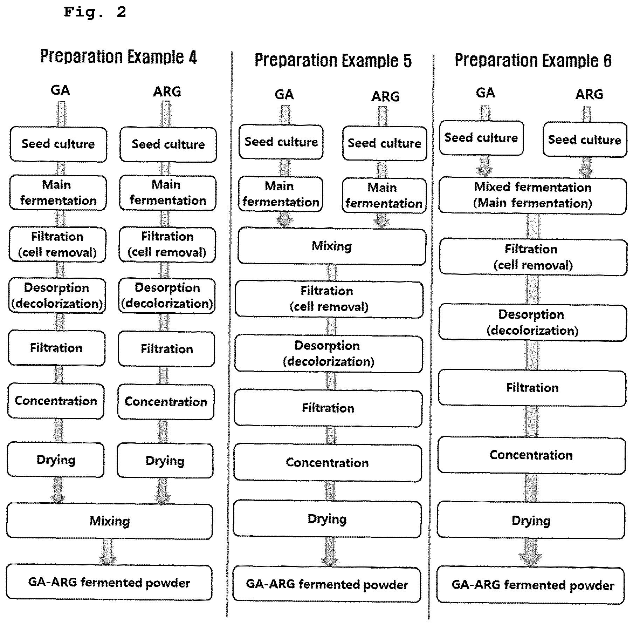

Conventionally, a flavor containing glutamic acid and arginine was produced by individually fermenting a glutamic acid-producing microorganism and an arginine-producing microorganism and then mixing the amino acid-containing fermentation broths or dried products thereof at a suitable ratio. To compare the differences in taste between this conventional individual fermentation method and the method based on mixed fermentation of the glutamic acid-producing microorganism and the arginine-producing microorganism, the components of glutamic acid-arginine (GA-ARG) fermented powders obtained by the production methods were compared (see FIG. 2).

The glutamic acid seed culture broth and arginine seed culture broth used here were prepared in the same manner as in Example 3-1.

### ① Individual fermentation (Preparation Example 4)

In the method of mixing individual dried products among the conventional methods, each of the glutamic acid seed culture broth and the arginine seed culture broth was transferred into a 50-L fermenter and then individually subjected to main fermentation. Next, cells were separated from each fermentation broth, and then decolorization and filtration processes were performed. The filtrates were concentrated and dried to obtain dried products. The obtained dried glutamic acid and arginine products were mixed together so that the ratio of glutamic acid to arginine was 1:1, thereby preparing fermented powder containing glutamic acid and arginine.

### ② Mixing of fermentation broths after individual fermentation (Preparation Example 5)

In the method of mixing individual fermentation broths among the conventional methods, each of the glutamic acid seed culture broth and the arginine seed culture broth was transferred into a 50-L fermenter and then individually subjected to main fermentation. The fermentation broths obtained in the main fermentation were mixed together so that the ratio of glutamic acid to arginine was 1: 1. Next, cells were separated from the fermentation broth mixture, and then decolorization and filtration processes were performed. The filtrate was concentrated and dried to obtain glutamic acid-arginine fermented powder.

### ③ Mixed fermentation (Preparation Example 6)

In mixed fermentation, the glutamic acid seed culture broth and the arginine seed culture broth were inoculated at a ratio of 30: 70 in the same manner as in Example 3-2 so that the ratio of glutamic acid to arginine in the fermentation broth was about 1:1 in the same manner as in Preparation Examples 4 and 5, followed by mixed fermentation. Next, cells were separated from the fermentation broth, and decolorization and filtration processes were performed. The filtrate was concentrated and dried to obtain glutamic acid-arginine fermented powder.

### 4-2. Comparison of components between glutamic acid-arginine fermented powders

Component analysis was performed on the glutamic acid-arginine fermented powder obtained through individual fermentation in each of Preparation Examples 4 and 5 and the glutamic acid-arginine fermented powder obtained through mixed fermentation in Preparation Example 6.

Amino acids (L-glutamic acid and L-arginine) were measured by HPLC analysis (GA - 210 nm, UV detector, flow rate 0.9 ml/min; ARG - 195 nm, UV detector, flow rate 1 ml/min). Organic acid and ions were measured in the same manner as in Example 2-2. The results are shown in Table 9 below.

**[Table 9]**

| Item | Preparation Example 4 | Preparation Example 5 | Preparation Example 6 |
|---|---|---|---|
| GA | 22% | 35% | 42% |
| ARG | 22% | 34% | 42% |
| AA/TS* | 48% | 71% | 86% |
| Total nitrogen | 9.4% | 12.0% | 11.7% |
| Organic acids | 3.6% | 2.5% | 1.8% |
| Ions | 8.3% | 9.9% | 2.1% |
| Ammonium | 4.0% | 2.6% | 0.9% |
| *AA/TS: proportion of amino acids (GA+ ARG) relative to total solid content | | | |

Referring to Table 9 above, mixed fermentation (Preparation Example 6) showed a higher amino acid proportion than individual fermentation (Preparation Examples 4 and 5) and showed significant decreases in the contents of organic acids, ions and ammonium.

### 4-3. Sensory comparison between individual fermentation and mixed fermentation

Sensory evaluation was performed on the glutamic acid-arginine fermented powders obtained through individual fermentation in Preparation Examples 4 and 5 and the glutamic acid-arginine fermented powder obtained through mixed fermentation in Preparation Example 6.

Sensory evaluation was performed in the same manner as in Example 2-3. The results are shown in Table 10 below.

**[Table 10]**

| Item | Preparation Example 4 (individual fermentation) | Preparation Example 5 (individual fermentation) | Preparation Example 6 (mixed fermentation) |
|---|---|---|---|
| Initial Umami taste | ++ | +++ | ++++ |
| Umami persistence | + | ++ | ++++ |
| Salty taste | - | + | ++ |
| Sour taste | + | + | - |
| Bitter taste | ++ | ++ | - |
| Sweet taste | - | - | - |

Referring to Table 10 above, due to the difference in components of the glutamic acid-arginine fermented powder as shown in Table 9 above, the sample prepared by the mixing process after individual fermentation showed a weak umami taste and an increased bitter taste compared to the sample prepared by mixed fermentation. In addition, since the increase in by-products such as organic acids and the increase in ions as shown in Table 9 above also affect the sensory properties of the sample, the mixed fermentation process is more effective in terms of taste or process simplification than mixing after individual fermentation.

### Example 5. Mixed fermentation of glutamic acid and inosinic acid

### 5-1. Seed culture

*Corynebacterium glutamicum* NFG6 (KCCM13164P) was used as a glutamic acid-producing microorganism, and *Corynebacterium ammoniagenes* NFI545 (KCCM13162P) that produces IMP was used as an inosinic acid-producing microorganism.

A glutamic acid seed culture broth of the glutamic acid-producing microorganism was prepared in the same manner as in Example 1-1.

For seed culture of the inosinic acid-producing microorganism, the inosinic acid-producing microorganism was inoculated into a 2-L flask containing 0.3 L of a seed culture medium, followed by primary culture for 20 to 24 hours at 31°C and 150 rpm (OD₆₁₀ = 15 to 20). Next, 1% of the primary culture was inoculated into a 5-L jar fermenter, and 2 to 2.5 L of a seed culture medium was added thereto, followed by secondary culture at 31°C, pH 7.1, 600 rpm and an aeration rate of 1.0 vvm for 21 to 24 hours (OD₆₁₀ = 20 to 40), thereby preparing an inosinic acid seed culture broth. The composition of the seed culture medium used is shown in Table 11 below.

**[Table 11]**

| | Composition |
|---|---|
| Seed culture medium for inosinic acid-producing microorganism | 4 to 6% glucose, 2 to 4% yeast extract paste, 0.3% (NH₄)₂SO₄, 0.2% KH₂PO₄, 0.2% K₂HPO₄, 200 to 300 ppm adenine, 200 to 300 ppm guanine, 0.15% MgSO₄·7H₂O, 10 ppm nicotinic acid, 100 ppm Capantothenate, 15 ppm cysteine, 1 ppm thiamine HCl, 5 ppm ZnSO₄·7H₂O, 10 ppm MnSO₄·5H₂O, 0.1 ppm biotin, 15 ppm FeSO₄·7H₂O, and 0.01% antifoaming agent for food |

### 5-2. Main fermentation

In order to examine the ratio of glutamic acid to inosinic acid in the fermentation broth depending on the inoculums of the glutamic acid seed culture broth and the inosinic acid seed culture broth, the glutamic acid seed culture broth and the inosinic acid seed culture broth were inoculated at various ratios and fermented.

In main fermentation, 14 to 18 L of a fermentation medium was added to a 50-L fermenter and inoculated with 1.2 to 1.8 L of the total seed culture broth at an inoculation ratio of 0.05 to 99.95 (glutamic acid seed culture broth): 99.95 to 0.05 (inosinic acid seed culture broth), followed by mixed fermentation by fed-batch culture for 30 to 90 hours. The fermentation medium composition and fermentation conditions used here are shown in Table 12 below.

**[Table 12]**

| | |
|---|---|
| Fermentation medium composition | 1 to 3% molasses, 5 to 7% raw sugar, 2 to 3% yeast extract paste, 0.6 to 1.2% H₃PO₄, 0.05 to 0.1% betaine, 100 to 200 ppm adenine, 50 to 150 ppm guanine, 0.2 to 0.5% MgSO₄·7H₂O, 50 to 100 ppm Ca-pantothenate, 5 to 15 ppm vitamin B3, 5 to 20 ppm thiamine HCl, 0.4 to 0.8% NaOH, 5 to 10 ppm FeSO₄, 10 to 20 ppm MnSO₄, 10 to 20 ppm ZnSO₄, and 0.005% antifoaming agent for food |
| Fermentation | Temperature 31 to 32°C, pH 6.5 to 7.5, aeration |
| conditions | rate 0.8 to 1.2 vvm, internal pressure 0.6 to 1.0 kg/cm³, agitation speed 320 to 350 rpm, and dissolved oxygen (DO) concentration 20 to 70% |

This mixed fermentation was performed a total of three times, the average value was calculated, and the results are shown in Table 13 below.

**[Table 13]**

| Inoculation ratio between seed culture broths (GA: IMP) | Fermentation time | GA: IMP ratio in fermentation broth |
|---|---|---|
| 99.95 : 0.05 | 33 hours | 99.8 : 1 |
| 80 : 20 | 42 hours | 51.3 : 1 |
| 70 : 30 | 48 hours | 31.2 : 1 |
| 50 : 50 | 60 hours | 16.8 : 1 |
| 20 : 80 | 83 hours | 1.04 : 1 |
| 0.05 : 99.95 | 90 hours | 0.02 : 1 |

Referring to Table 13 above, it was confirmed that, when the seed culture broths of the microorganisms were inoculated at a ratio of 0.05 to 99.95 (glutamic acid-producing microorganism): 99.95 to 0.05 (inosinic acid-producing microorganism), L-glutamic acid and IMP in the fermentation broth were produced at a ratio of 0.02 to 99.8: 1.

### Example 6. Comparison of glutamic acid-inosinic acid fermented powders between fermentation processes

### 6-1. Production of glutamic acid-inosinic acid fermented powder

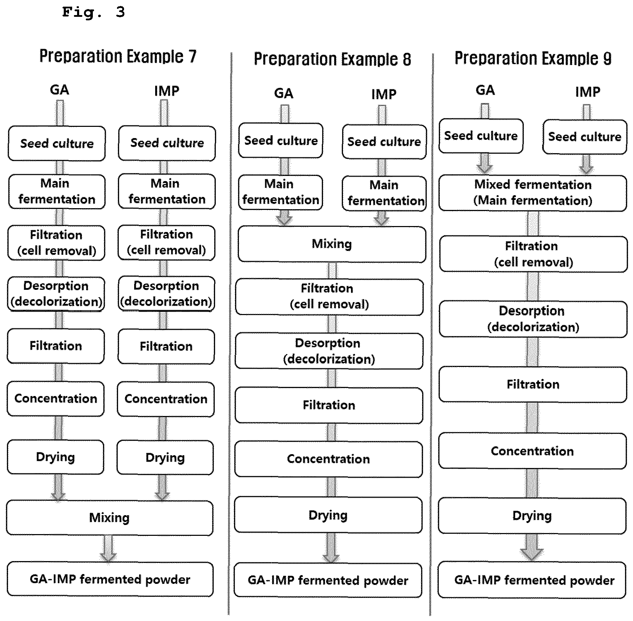

Conventionally, a flavor containing glutamic acid and inosinic acid was produced by individually fermenting a glutamic acid-producing microorganism and an inosinic acid-producing microorganism and then mixing the fermentation broths or dried products thereof at a suitable ratio. To compare the differences in taste between this conventional individual fermentation method and the method based on mixed fermentation of the glutamic acid-producing microorganism and the inosinic acid-producing microorganism, the components of glutamic acid-inosinic acid (GA-IMP) fermented powders obtained by the production methods were compared (see FIG. 3).

The glutamic acid seed culture broth and inosinic acid seed culture broth used here were prepared in the same manner as in Example 5-1.

### ① Individual fermentation (Preparation Example 7)

In the method of mixing individual dried products among the conventional methods, each of the glutamic acid seed culture broth and the inosinic acid seed culture broth was transferred into a 50-L fermenter and then individually subjected to main fermentation. Next, cells were separated from each fermentation broth, and then decolorization and filtration processes were performed. The filtrates were concentrated and dried to obtain dried products. The obtained dried glutamic acid and isosinic acid products were mixed together so that the ratio of glutamic acid to inosinic acid was 1:1, thereby preparing fermented powder containing glutamic acid and inosinic acid.

### ② Mixing of fermentation broths after individual fermentation (Preparation Example 8)

In the method of mixing individual fermentation broths among the conventional methods, each of the glutamic acid seed culture broth and the inosinic acid seed culture broth was transferred into a 50-L fermenter and then individually subjected to main fermentation. The fermentation broths obtained in the main fermentation were mixed together so that the ratio of glutamic acid to inosinic acid was 1: 1. Next, cells were separated from the fermentation broth mixture, and then decolorization and filtration processes were performed. The filtrate was concentrated and dried to obtain glutamic acid-inosinic acid fermented powder.

### ③ Mixed fermentation (Preparation Example 9)

In mixed fermentation, the glutamic acid seed culture broth and the inosinic acid seed culture broth were inoculated at a ratio of 20: 80 in the same manner as in Example 5-2 so that the ratio of glutamic acid to inosinic acid in the fermentation broth was about 1:1 in the same manner as in Preparation Examples 7 and 8, followed by mixed fermentation. Next, cells were separated from the fermentation broth, and decolorization and filtration processes were performed. The filtrate was concentrated and dried to obtain glutamic acid-inosinic acid fermented powder.

### 6-2. Comparison of components between glutamic acid-inosinic acid fermented powders

Component analysis was performed on the glutamic acid-inosinic acid fermented powder obtained through individual fermentation in each of Preparation Examples 7 and 8 and the glutamic acid-inosinic acid fermented powder obtained through mixed fermentation in Preparation Example 9.

L-glutamic acid and IMP were measured by HPLC analysis (GA - 210 nm, UV detector, flow rate 0.9 ml/min; IMP - 254 nm, UV detector, flow rate 0.9 ml/min). Organic acid and ions were measured in the same manner as in Example 2-2. The results are shown in Table 14 below.

**[Table 14]**

| Item | Preparation Example 7 | Preparation Example 8 | Preparation Example 9 |
|---|---|---|---|
| GA | 22% | 31% | 37% |
| IMP | 20% | 31% | 36% |
| (GA+IMP)/TS* | 42% | 62% | 73% |
| Total nitrogen | 8.6% | 9.7% | 10.5% |
| Organic acids | 4.0% | 3.3% | 3.1% |
| Ions | 10.4% | 8.8% | 4.2% |
| Ammonium | 4.0% | 3.0% | 1.7% |
| *(GA+IMP)/TS: Proportion of product relative to total solid content | | | |

### 6-3. Sensory comparison between individual fermentation and mixed fermentation

Sensory evaluation was performed on the glutamic acid-inosinic acid fermented powders obtained through individual fermentation in Preparation Examples 7 and 8 and the glutamic acid-inosinic acid fermented powder obtained through mixed fermentation in Preparation Example 9.

Sensory evaluation was performed in the same manner as in Example 2-3. The results are shown in Table 15 below.

**[Table 15]**

| Item | Preparation Example 7 (indi vidual fermentation) | Preparation Example 8 (indi vidual fermentation) | Preparation Example 9 (mixed fermentation) |
|---|---|---|---|
| Initial Umami taste | +++ | ++++ | ++++ |
| Umami persistence | + | +++ | ++++ |
| Kokumi | + | ++ | +++ |
| Salty taste | + | ++ | ++ |
| Sour taste | ++ | ++ | + |
| Bitter taste | ++ | + | - |
| Sweet taste | + | + | ++ |

Referring to Table 15 above, due to the difference in components of the glutamic acid-inosinic acid fermented powder as in Table 14 above, the sample prepared by the mixing process after individual fermentation showed a weak umami taste and umami persistence and an increased bitter taste compared to the sample prepared by mixed fermentation. In addition, since the increase in by-products such as organic acids and the increase in ions as shown in Table 14 above also affect the sensory properties of the sample, the mixed fermentation process is more effective in terms of taste or process simplification than mixing after individual fermentation.

### Example 7. Mixed fermentation of inosinic acid and lysine

### 7-1. Seed culture

*Corynebacterium ammoniagenes* NFI545 (KCCM13162P) was used as an inosinic acid-producing microorganism, and *Corynebacterium glutamicum* NFL21 (KCCM13163P) was used as a lysine-producing microorganism.

An inosinic acid seed culture broth of the inosinic acid-producing microorganism and a lysine seed culture broth of the lysine-producing microorganism were prepared in the same manner as in Examples 5-1 and 1-1, respectively.

### 7-2. Main fermentation

In order to examine the ratio of inosinic acid to lysine in the fermentation broth depending on the inoculums of the inosinic acid seed culture broth and the lysine seed culture broth, the inosinic acid seed culture broth and the lysine seed culture broth were inoculated at various ratios and fermented.

In main fermentation, 14 to 18 L of a fermentation medium was added to a 50-L fermenter and inoculated with 1.2 to 1.8 L of the total seed culture broth at an inoculation ratio of 0.05 to 99.95 (inosinic acid seed culture broth): 99.95 to 0.05 (lysine seed culture broth), followed by mixed fermentation by fed-batch culture for 45 to 90 hours. The fermentation medium composition and fermentation conditions used here are shown in Table 16 below.

**[Table 16]**

| | |
|---|---|
| Fermentation medium composition | 1 to 3% molasses, 5 to 7% raw sugar, 2 to 3% yeast extract paste, 0.6 to 1.2% H₃PO₄, 0.05 to 0.1% betaine, 100 to 200 ppm adenine, 50 to 150 ppm guanine, 0.2 to |
| | 0.5% MgSO₄·7H₂O, 50 to 100 ppm Ca-pantothenate, 5 to 15 ppm vitamin B3, 5 to 20 ppm thiamine HCl, 0.4% (NH₄)₂SO₄, 0.4 to 0.8% NaOH, 5 to 10 ppm FeSO₄, 10 to 20 ppm MnSO₄, 10 to 20 ppm ZnSO₄, and 0.005% antifoaming agent for food |
| Fermentation conditions | Temperature 31 to 32°C, pH 6.5 to 7.5, aeration rate 0.8 to 1.2 vvm, internal pressure 0.6 to 1.0 kg/cm³, agitation speed 320 to 350 rpm, and dissolved oxygen (DO) concentration 20 to 70% |

This mixed fermentation was performed a total of three times, the average value was calculated, and the results are shown in Table 17 below.

**[Table 17]**

| Inoculation ratio between seed culture broths (IMP: LYS) | Fermentation time | IMP: LYS ratio in fermentation ratio |
|---|---|---|
| 99.95 : 0.05 | 90 hours | 92.3 : 1 |
| 80 : 20 | 73 hours | 1.01 : 1 |
| 50 : 50 | 61 hours | 0.05 : 1 |
| 20 : 80 | 48 hours | 0.02 : 1 |
| 0.05 : 99.95 | 45 hours | 0.01 : 1 |

Referring to Table 17 above, it was confirmed that, when the seed culture broths of the microorganisms were inoculated at a ratio of 0.05 to 99.95 (inosinic acid-producing microorganism): 99.95 to 0.05 (lysine-producing microorganism), IMP and lysine in the fermentation broth were produced at a ratio of 0.01 to 92: 1.

### Example 8. Comparison of inosinic acid-lysine fermented powders between fermentation processes

### 8-1. Production of inosinic acid-lysine fermented powder

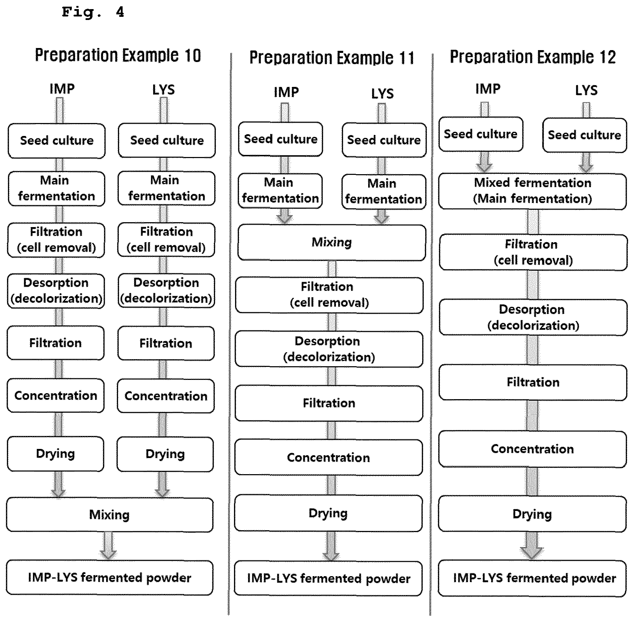

Conventionally, a flavor containing inosinic acid and lysine was produced by individually fermenting an inosinic acid-producing microorganism and a lysine-producing microorganism and then mixing the fermentation broths or dried products thereof at a suitable ratio. To compare the differences in taste between this conventional individual fermentation method and the method based on mixed fermentation of the inosinic acid-producing microorganism and the lysine-producing microorganism, the components of inosinic acid-lysine (IMP-LYS) fermented powders obtained by the production methods were compared (see FIG. 4).

The inosinic acid seed culture broth and lysine seed culture broth used here were prepared in the same manner as in Example 7-1.

### ① Individual fermentation (Preparation Example 10)

In the method of mixing individual dried products among the conventional methods, each of the inosinic acid seed culture broth and the lysine seed culture broth was transferred into a 50-L fermenter and then individually subjected to main fermentation. Next, cells were separated from each fermentation broth, and then decolorization and filtration processes were performed. The filtrates were concentrated and dried to obtain dried products. The obtained dried inosinic acid and lysine products were mixed together so that the ratio of inosinic acid to lysine was 1:1, thereby preparing fermented powder containing inosinic acid and lysine.

### ② Mixing of fermentation broths after individual fermentation (Preparation Example 11)

In the method of mixing individual fermentation broths among the conventional methods, each of the inosinic acid seed culture broth and the lysine seed culture broth was transferred into a 50-L fermenter and then individually subjected to main fermentation. The fermentation broths obtained in the main fermentation were mixed together so that the ratio of inosinic acid to lysine was 1: 1. Next, cells were separated from the fermentation culture broth, and then decolorization and filtration processes were performed. The filtrate was concentrated and dried to obtain inosinic acid-lysine fermented powder.

### ③ Mixed fermentation (Preparation Example 12)

In mixed fermentation, the inosinic acid seed culture broth and the lysine seed culture broth were inoculated at a ratio of 80: 20 in the same manner as in Example 7-2 so that the ratio of inosinic acid to lysine in the fermentation broth was about 1:1 in the same manner as in Preparation Examples 10 and 11, followed by mixed fermentation. Next, cells were separated from the fermentation broth, and decolorization and filtration processes were performed. The filtrate was concentrated and dried to obtain inosinic acid-lysine fermented powder.

### 8-2. Comparison of components between lysine-inosinic acid fermented powders

Component analysis was performed on the inosinic acid-lysine fermented powder obtained through individual fermentation in each of Preparation Examples 10 and 11 and the inosinic acid-lysine fermented powder obtained through mixed fermentation in Preparation Example 12.

IMP and L-lysine were measured by HPLC analysis (IMP - 254 nm, UV detector, flow rate 0.9 ml/min; LYS - 214 nm, UV detector, flow rate 0.8 ml/min). Organic acid and ions were measured in the same manner as in Example 2-2. The results are shown in Table 18 below.

**[Table 18]**

| Item | Preparation Example 10 | Preparation Example 11 | Preparation Example 12 |
|---|---|---|---|
| LYS | 26% | 32% | 38% |
| IMP | 24% | 30% | 33% |
| (LYS+IMP)/TS* | 45% | 62% | 71% |
| Total nitrogen | 9.7% | 11.3% | 12.5% |
| Organic acids | 5.0% | 4.1% | 3.7% |
| Ions | 9.2% | 8.2% | 5.6% |
| Ammonium | 3.9% | 3.5% | 1.9% |
| *(LYS+IMP)/TS: proportion of product relative to total solid | | | |

**Referring** to Table 18 above, mixed fermentation (Preparation Example 12) showed a higher LYS+IMP proportion than individual fermentation (Preparation Examples 10 and 11) and showed significant decreases in the contents of organic acids, ions and ammonium.

### 8-3. Sensory comparison between individual fermentation and mixed fermentation

Sensory evaluation was performed on the inosinic acid-lysine fermented powders obtained through individual fermentation in Preparation Examples 10 and 11 and the inosinic acid-lysine fermented powder obtained through mixed fermentation in Preparation Example 12.

Sensory evaluation was performed in the same manner as in Example 2-3. The results are shown in Table 19 below.

**[Table 19]**

| Item | Preparation Example 10 (individual fermentation) | Preparation Example 11 (individual fermentation) | Preparation Example 12 (mixed fermentation) |
|---|---|---|---|
| Initial Umami taste | + | ++ | ++ |
| Umami persistence | + | +++ | ++++ |
| Kokumi | + | ++ | +++ |
| Salty taste | + | ++ | ++ |
| Sour taste | ++ | ++ | + |
| Bitter taste | ++ | ++ | - |
| Sweet taste | + | + | ++ |

Referring to Table 19 above, due to the difference in components of the inosinic acid-lysine fermented powder as shown in Table 18 above, the sample prepared by the mixing process after individual fermentation showed weak umami persistence and an increased bitter taste compared to the sample prepared by mixed fermentation. In addition, since the increase in by-products such as organic acids and the increase in ions as shown in Table 18 above also affect the sensory properties of the sample, the mixed fermentation process is more effective in terms of taste or process simplification than mixing after individual fermentation.

### Example 9. Mixed fermentation of inosinic acid and arginine

### 7-1. Seed culture

*Corynebacterium ammoniagenes* NFI545 (KCCM13162P) was used as an inosinic acid-producing microorganism, and *Corynebacterium glutamicum* NFA40 (KCCM13165P) was used as an arginine-producing microorganism.

An inosinic acid seed culture broth of the inosinic acid-producing microorganism and an arginine seed culture broth of the arginine-producing microorganism were prepared in the same manner as in Examples 5-1 and 3-1, respectively.

### 9-2. Main fermentation

In order to examine the ratio of inosinic acid to arginine in the fermentation broth depending on the inoculums of the inosinic acid seed culture broth and the arginine seed culture broth, the inosinic acid seed culture broth and the arginine seed culture broth were inoculated at various ratios and fermented.

In main fermentation, 14 to 18 L of a fermentation medium was added to a 50-L fermenter and inoculated with 1.2 to 1.8 L of the total seed culture broth at an inoculation ratio of 65 to 99.95 (inosinic acid seed culture broth): 35 to 0.05 (arginine seed culture broth), followed by mixed fermentation by fed-batch culture for 45 to 80 hours. The fermentation medium composition and fermentation conditions used here are shown in Table 20 below.

**[Table 20]**

| | |
|---|---|
| Fermentation medium composition | 1 to 3% molasses, 5 to 7% raw sugar, 2 to 3% yeast extract paste, 0.6 to 1.2% H₃PO₄, 0.05 to 0.1% betaine, 100 to 200 ppm adenine, 50 to 150 ppm guanine, 0.2 to 0.5% MgSO₄·7H₂O, 50 to 100 ppm Ca-pantothenate, 5 to 15 ppm vitamin B3, 5 to 20 ppm thiamine HCl, 0.4% (NH₄)₂SO₄, 0.4 to 0.8% NaOH, 5 to 10 ppm FeSO₄, 10 to 20 ppm MnSO₄, 10 to 20 ppm ZnSO₄, and 0.005% antifoaming agent for food |
| Fermentation conditions | Temperature 31 to 32°C, pH 6.5 to 7.5, aeration rate 0.8 to 1.2 vvm, internal pressure 0.6 to 1.0 kg/cm³, agitation speed 320 to 350 rpm, and dissolved oxygen (DO) concentration 20 to 70% |

This mixed fermentation was performed a total of three times, the average value was calculated, and the results are shown in Table 21 below.

**[Table 21]**

| Inoculation ratio between seed culture broths (IMP: ARG) | Fermentation time | IMP: ARG ratio in fermentation broth |
|---|---|---|
| 99.95 : 0.05 | 80 hours | 95.1 : 1 |
| 80 : 20 | 46 hours | 5.3 : 1 |
| 70 : 30 | 45 hours | 2.45 : 1 |
| 65 : 35 | 45 hours | 1.04 : 1 |

Referring to Table 21 above, it was confirmed that, when the seed culture broths of the microorganisms were inoculated at a ratio of 65 to 99.95 (inosinic acid-producing microorganism): 35 to 0.05 (arginine-producing microorganism), IMP and L-arginine in the fermentation broth were produced at a ratio of 1.04 to 95.1: 1.

### Example 10. Comparison of inosinic acid-arginine fermented powders between fermentation processes

### 10-1. Production of inosinic acid-arginine fermented powder

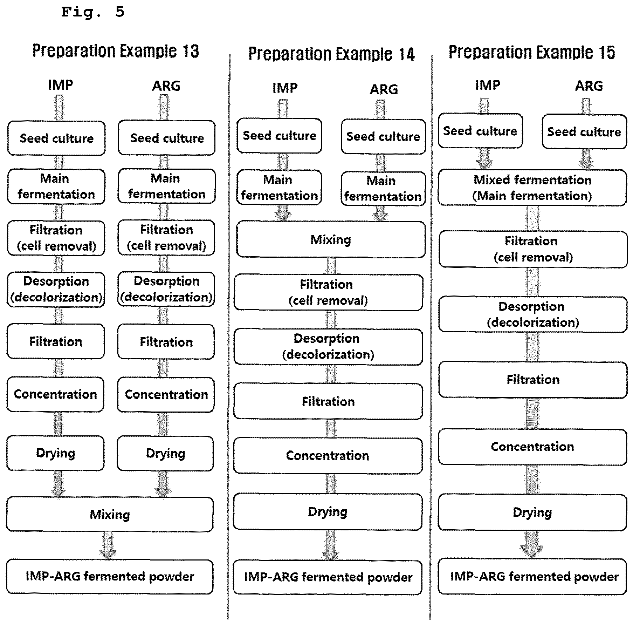

Conventionally, a flavor containing inosinic acid and arginine was produced by individually fermenting an inosinic acid-producing microorganism and an arginine-producing microorganism and then mixing the fermentation broths or dried products thereof at a suitable ratio. To compare the differences in taste between this conventional individual fermentation method and the method based on mixed fermentation of the inosinic acid-producing microorganism and the arginine-producing microorganism, the components of inosinic acid-arginine (IMP-ARG) fermented powders obtained by the production methods were compared (see FIG. 5).

The inosinic acid seed culture broth and arginine seed culture broth used here were prepared in the same manner as in Example 9-1.

### ① Individual fermentation (Preparation Example 13)

In the method of mixing individual dried products among the conventional methods, each of the inosinic acid seed culture broth and the arginine seed culture broth was transferred into a 50-L fermenter and then individually subjected to main fermentation. Next, cells were separated from each fermentation broth, and then decolorization and filtration processes were performed. The filtrates were concentrated and dried to obtain dried products. The obtained dried inosinic acid and arginine products were mixed together so that the ratio of inosinic acid to arginine was 1:1, thereby preparing fermented powder containing inosinic acid and arginine.

### ② Mixing of fermentation broths after individual fermentation (Preparation Example 14)

In the method of mixing individual fermentation broths among the conventional methods, each of the inosinic acid seed culture broth and the arginine seed culture broth was transferred into a 50-L fermenter and then individually subjected to main fermentation. The fermentation broths obtained in the main fermentation were mixed together so that the ratio of inosinic acid to arginine was 1: 1. Next, cells were separated from the fermentation broth mixture, and then decolorization and filtration processes were performed. The filtrate was concentrated and dried to obtain inosinic acid-arginine fermented powder.

### ③ Mixed fermentation (Preparation Example 15)

In mixed fermentation, the inosinic acid seed culture broth and the arginine seed culture broth were inoculated at a ratio of 65: 35 in the same manner as in Example 9-2 so that the ratio of inosinic acid to arginine in the fermentation broth was about 1:1 in the same manner as in Preparation Examples 13 and 14, followed by mixed fermentation. Next, cells were separated from the fermentation broth, and decolorization and filtration processes were performed. The filtrate was concentrated and dried to obtain inosinic acid-arginine fermented powder.

### 10-2. Comparison of components between inosinic acid-arginine fermented powders

Component analysis was performed on the inosinic acid-arginine fermented powder obtained through individual fermentation in each of Preparation Examples 13 and 14 and the inosinic acid-arginine fermented powder obtained through mixed fermentation in Preparation Example 15.

L-arginine and IMP were measured by HPLC analysis (ARG - 195 nm, UV detector, flow rate 1 ml/min; IMP - 254 nm, UV detector, flow rate 0.9 ml/min). Organic acid and ions were measured in the same manner as in Example 2-2. The results are shown in Table 22 below.

**[Table 22]**

| Item | Preparation Example 13 | Preparation Example 14 | Preparation Example 15 |
|---|---|---|---|
| ARG | 24% | 31% | 34% |
| IMP | 24% | 31% | 33% |
| (ARG+IMP) /TS* | 48% | 62% | 76% |
| Total nitrogen | 9.7% | 11.3% | 14.1% |
| Organic acids | 4.7% | 3.8% | 2.5% |
| Ions | 7.5% | 6.2% | 3.8% |
| Ammonium | 3.1% | 2.5% | 0.8% |
| *(ARG+IMP)/TS: Proportion of product relative to total solid content | | | |

### 10-3. Sensory comparison between individual fermentation and mixed fermentation

Sensory evaluation was performed on the inosinic acid-arginine fermented powders obtained through individual fermentation in Preparation Examples 13 and 14 and the inosinic acid-arginine fermented powder obtained through mixed fermentation in Preparation Example 15.

Sensory evaluation was performed in the same manner as in Example 2-3. The results are shown in Table 23 below.

**[Table 23]**

| Item | Preparation Example 13 (individual fermentation) | Preparation Example 14 (individual fermentation) | Preparation Example 15 (mixed fermentation) |
|---|---|---|---|
| Initial Umami taste | + | ++ | ++ |
| Umami persistence | + | +++ | ++++ |
| Kokumi | + | ++ | +++ |
| Salty taste | + | ++ | ++ |
| Sour taste | ++ | ++ | + |
| Bitter taste | ++ | ++ | - |
| Sweet taste | - | - | - |

Referring to Table 23 above, due to the difference in components of the inosinic acid-arginine fermented powder as shown in Table 22 above, the sample prepared by the mixing process after individual fermentation showed weak umami persistence and an increased bitter taste compared to the sample prepared by mixed fermentation. In addition, since the increase in by-products such as organic acids and the increase in ions as shown in Table 22 above also affect the sensory properties of the sample, the mixed fermentation process is more effective in terms of taste or process simplification than mixing after individual fermentation.

So far, the present invention has been described with reference to the preferred embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the scope of the present invention.

### [Accession Numbers]

Depository authority: Korean Culture Center of Microorganisms (KCCM)
Accession number: KCCM13162P
Deposit date: April 21, 2022
Depository authority: Korean Culture Center of Microorganisms (KCCM)
Accession number: KCCM13163P
Deposit date: April 21, 2022
Depository authority: Korean Culture Center of Microorganisms (KCCM)
Accession number: KCCM13164P
Deposit date: April 21, 2022
Depository authority: Korean Culture Center of Microorganisms (KCCM)
Accession number: KCCM13165P
Deposit date: April 21, 2022

## Claims

1. A method for producing a flavor, the method comprising a step of inoculating a fermentation medium with a first microorganism and a second microorganism and then producing a fermentation broth containing amino acid, nucleic acid and/or organic acid by fermentation of the microorganisms,
wherein the first microorganism and the second microorganism produce different products and each produces one selected from the group consisting of amino acid, nucleic acid and organic acid.

2. The method of claim 1, wherein the amino acid is at least one selected from the group consisting of L-glutamic acid, L-alanine, L-valine, L-leucine, L-isoleucine, L-proline, L-phenylalanine, L-tryptophan, L-methionine, L-glycine, L-serine, L-threonine, L-cysteine, L-asparagine, L-glutamine, L-aspartic acid, L-lysine, L-arginine, and L-histidine.

3. The method of claim 1 or 2, wherein the nucleic acid is at least one selected from the group consisting of inosinic acid, guanylic acid, xanthylic acid, and salts thereof.

4. The method according to any one of claims 1 to 3, wherein the organic acid is at least one selected from the group consisting of succinic acid, malic acid, citric acid, acetic acid, lactic acid, fumaric acid, tartaric acid, ascorbic acid, gluconic acid, and salts thereof.

5. The method according to any one of claims 1 to 4, wherein the first microorganism is a glutamic acid-producing microorganism, and the second microorganism is a lysine-producing microorganism, an arginine-producing microorganism, a histidine-producing microorganism, a tryptophan-producing microorganism, a glycine-producing microorganism, an alanine-producing microorganism, a succinic acid-producing microorganism, a lactic acid-producing microorganism, a guanylic acid-producing microorganism, or an inosinic acid-producing microorganism.

6. The method according to any one of claims 1 to 4, wherein the first microorganism is an inosinic acid-producing microorganism, and the second microorganism is a lysine-producing microorganism, an arginine-producing microorganism, a histidine-producing microorganism, a tryptophan-producing microorganism, a glycine-producing microorganism, an alanine-producing microorganism, a succinic acid-producing microorganism, a lactic acid-producing microorganism, or a guanylic acid-producing microorganism.

7. The method according to any one of claims 1 to 6, wherein the step comprises further inoculating the fermentation medium with a third microorganism, which produces a product different from products produced from the first microorganism and the second microorganism and produces one selected from the group consisting of amino acid, nucleic acid and organic acid.

8. The method according to any one of claims 1 to 5 and 7, wherein the first microorganism is a glutamic acid-producing microorganism, the second microorganism is a guanylic acid-producing microorganism, the third microorganism is an inosinic acid-producing microorganism.

9. The method according to any one of claims 1 to 8, wherein the first microorganism, the second microorganism and the third microorganism are microorganisms of the genus *Corynebacterium.*

10. The method according to any one of claims 1 to 9, wherein the first microorganism, the second microorganism and the third microorganism are in a seed culture broth state obtained by individual culture or co-culture.

11. The method according to any one of claims 1 to 10, wherein the step comprises adjusting the inoculum of each microorganism in order to control the ratio between the products of the microorganisms in the fermentation broth.

12. The method according to any one of claims 1 to 11, wherein the fermentation broth contains the products of the microorganisms in an amount of 3 to 90 wt% relative to the total solid content of the fermentation broth.

13. A method for producing a flavor containing L-glutamic acid and L-lysine, the method comprising a step of inoculating a fermentation medium with a glutamic acid-producing microorganism and a lysine-producing microorganism and then producing a fermentation broth containing L-glutamic acid and L-lysine by fermentation of the microorganisms.

14. The method of claim 13, wherein the glutamic acid-producing microorganism and the lysine-producing microorganism are microorganisms of the genus *Corynebacterium.*

15. The method of claim 13 or 14, wherein the glutamic acid-producing microorganism and the lysine-producing microorganism are in a seed culture broth state obtained by individual culture or co-culture.

16. The method according to any one of claims 13 to 15, wherein the step comprises adjusting the inoculum of each of the glutamic acid-producing microorganism and the lysine-producing microorganism in order to control the ratio of L-glutamic acid to L-lysine in the fermentation broth.

17. The method according to any one of claims 13 to 16, wherein the fermentation broth contains amino acids, including L-glutamic acid and L-lysine, in an amount of 3 to 90 wt% relative to the total solid content of the fermentation broth.

18. A flavor produced by the method according to any one of claims 1 to 12.

19. A flavor containing L-glutamic acid and L-lysine, produced by the method according to any one of claims 13 to 17.

20. The flavor of claim 19, containing L-glutamic acid and L-lysine in an amount of 3 to 90 wt% relative to the total solid content of the flavor.

21. A food composition containing the flavor of claim 18.

22. A food composition containing the flavor containing L-glutamic acid and L-lysine according to claim 19 or 20.
